# EUROPÄISCHE PATENTSCHRIFT

(11) **EP 1 697 048 B1**
(45) Veröffentlichungstag und Bekanntmachung des Hinweises auf die Patenterteilung: **21.08.2013**
(21) Anmeldenummer: 04803769.1
(22) Anmeldetag: 11.12.2004
(51) Int. Cl.: G01N 33/487

(54) **Analysehandgerät**
Hand-held analysis device
Appareil manuel d'analyse

(30) Priorität: 24.12.2003 DE 10361261
(43) Veröffentlichungstag der Anmeldung: 06.09.2006
(73) Patentinhaber: Roche Diagnostics GmbH, 68305 Mannheim (DE); F. Hoffmann-La Roche AG, 4070 Basel (CH)
(72) Erfinder: SCHULAT, Jochen, 68167 Mannheim (DE); SCHERER, Jörg, 4528 Zuchwill (CH); RASCH-MENGES, Jürgen, 68723 Schwetzingen (DE); MÜLLER, Josef, CH-9533 Kirchberg (CH); JANSEN, Paul, 68163 Mannheim (DE)
(74) Vertreter: Jany und Petersen
(86) Internationale Anmeldenummer: PCT/EP2004/014129
(87) Internationale Veröffentlichungsnummer: WO 2005/065828

(56) Entgegenhaltungen:
- EP-A- 0 742 436
- EP-A- 1 285 695
- EP-A- 1 286 162
- WO-A-01/23885
- US-A1- 2003 039 584
- US-A1- 2003 059 350

## Beschreibung

Die Erfindung betrifft ein Analysehandgerät zum Untersuchen einer Probe, insbesondere einer biologischen Flüssigkeit hinsichtlich eines medizinisch bedeutsamen Bestandteils, umfassend eine Analyseeinrichtung, eine Anzeigeeinrichtung, ein Gehäuse, das eine Ladeöffnung zum Aufnehmen eines auswechselbaren Magazins mit mehreren Kammern aufweist, die analytische Verbrauchsmittel, insbesondere Teststreifen, enthalten können und jeweils eine Öffnung an einer Stirnseite des Magazins aufweisen, die jeweils mit einer Siegelfolie verschlossen sein kann, eine Entnahmeeinrichtung zum Entnehmen eines der analytischen Verbrauchsmittel aus dem Magazin, mittels der eines der Verbrauchsmittel aus einer der Kammern des Magazins herausbefördert werden kann, wobei die von der Siegelfolie verschlossene Öffnung geöffnet wird, und einen Antrieb, der zum Positionieren einer der Kammern in eine Entnahmeposition, in der mittels der Entnahmeeinrichtung ein Verbrauchsmittel aus der Kammer entnehmbar ist, eine Bewegung des Magazins ermöglicht.

Für die chemische und biochemische Analyse von festen und flüssigen Probenmaterialien haben sich in darauf spezialisierten Labors und insbesondere auch für den Einsatz außerhalb fester Labors trägergebundene Schnelltests etabliert. Solche trägergebundene Schnelltests basieren auf einer eigens entwickelten Trockenchemie und sind trotz der oftmals komplexen Reaktion unter Beteiligung empfindlicher Reagenzien selbst von Laien einfach und unkompliziert durchzuführen.

Ein bekanntes Beispiel für trägergebundene Schnelltests sind Testelemente für die Bestimmung des Blutglucosegehalts bei Diabetikern. Diagnostische Testelemente, die streifenförmig ausgebildet sind, werden auch als Teststreifen bezeichnet. Bekannte Ausführungsformen sind z.B. Einfeld- oder Mehrfelder-Teststreifen für die Urinanalytik und diverse Indikatorpapiere. Da neben Testelementen in Streifenform auch andere Formen trägergebundener Tests existieren, spricht man allgemeiner von analytischen Verbrauchsmitteln, wozu beispielsweise auch Lanzetten oder Probeentnahmeelemente zählen.

Derartige analytische Verbrauchsmittel werden in einem Analysehandgerät verwendet, das beispielsweise mit einer optischen Analyseeinrichtung eine Verfärbung eines Teststreifens photometrisch auswertet. Die analytischen Verbrauchsmittel sind in einem Trommelmagazin gelagert, wie es beispielsweise in der EP 1 022 565 A2 beschrieben ist. Ein solches Trommelmagazin weist mehrere ringförmig angeordnete Kammern auf, die analytische Verbrauchsmittel enthalten können. Die Kammern weisen jeweils eine Einschuböffnung und eine Entnahmeöffnung an gegenüberliegenden Stirnseiten des Trommelmagazins auf. Diese Öffnungen sind jeweils mit einer Siegelfolie verschlossen, um die analytischen Verbrauchsmittel vor schädlichen Umwelteinflüssen, wie z.B. Licht, Feuchtigkeit oder Staub, zu schützen.

Bei bekannten Analysehandgeräten, beispielsweise dem "Accu-Chek® Compact Blutzuckermeßgerät" (Gebrauchsanweisung Publ. Nr. 3273571 (67)-06/01, Roche Diagnostics GmbH, Mannheim, 2001) wird durch Betätigung einer Entnahmeeinrichtung geprüft, ob sich in einer Kammer des Trommelmagazins noch ein analytisches Verbrauchsmittel befindet oder dieses bereits entnommen wurde. Das bekannte Analysehandgerät hat zwei Prüfstromkreise. Ein erster Prüfstromkreis wird von einem an einer Stößelstange der Entnahmeeinrichtung angebrachten Mitnehmer geschlossen, wenn diese Stößelstange in eine Kammer des Trommelmagazins eingeführt wird. Befindet sich in der Kammer ein Verbrauchsmittel, so wird es von der Stößelstange herausgeschoben und dabei von dem Verbrauchsmittel ein Schalter betätigt, der einen zweiten Prüfstromkreis schließt. Führt ein Betätigen der Entnahmeeinrichtung also nur zu einem Schließen des ersten Prüfstromkreises, nicht aber zum Schließen des zweiten Prüfstromkreises, so bedeutet dies, daß die betreffende Kammer des Trommelmagazins leer ist.

Analysehandgeräte zur Untersuchung eines medizinisch bedeutsamen Bestandteils einer Probe, wie beispielsweise Geräte zur Bestimmung des Blutglucosegehaltes, werden häufig von Personen verwendet, deren Wahrnehmung oder manuelle Geschicklichkeit wegen Krankheit oder Alter eingeschränkt ist. Es ist deshalb wichtig, daß sich solche Analysegeräte möglichst leicht handhaben lassen und Fehlbedienungen möglichst ausgeschlossen sind.

Wichtig ist es auch, daß tragbare Analysehandgeräte einen möglichst geringen Stromverbrauch aufweisen. Je niedriger nämlich der Stromverbrauch ist, desto seltener müssen Batterien ausgetauscht oder Akkus wieder aufgeladen werden, die die Stromquelle des Gerätes bilden. Bei einem ausreichend niedrigen Stromverbrauch des Gerätes ist auch eine Stromquelle in Form von Solarzellen möglich. Insgesamt müssen tragbare Analysehandgeräte also im Unterschied zu stationären Laborgeräten, die von technisch geschultem Personal bedient werden, möglichst leicht handhabbar sein, einen geringen Stromverbrauch aufweisen und ebenso wie die zugehörigen Magazine darauf angelegt sein, ständig von einem Benutzer mitgeführt zu werden.

Aufgabe der Erfindung ist es daher, einen Weg aufzuzeigen, wie bei einem Analysehandgerät die Handhabung erleichtert, die Zuverlässigkeit einer Untersuchung erhöht und der Stromverbrauch gesenkt werden kann.

Diese Aufgabe wird bei einem Analysehandgerät der eingangs genannten Art erfindungsgemäß dadurch gelöst, daß das Analysehandgerät eine Prüfeinrichtung aufweist, mit der ein Signal erzeugbar ist, das eine Information darüber enthält, ob die Öffnung einer der Kammern mit Siegelfolie verschlossen ist, wobei die Sensoreinrichtung mit einer Auswerteeinrichtung zum Auswerten des Signals verbunden ist.

Bei einem erfindungsgemäßen Analysehandgerät kann mittels der Prüfeinrichtung festgestellt werden, ob eine Öffnung einer Kammer mit Siegelfolie verschlossen ist, die Kammer also ein funktionsfähiges Verbrauchsmittel enthält. Die Öffnung ist sowohl nach einer Entnahme eines Verbrauchsmittels, als auch nach einer - beispielsweise durch unachtsamen Transport erfolgten - Beschädigung der Siegelfolie nicht mehr verschlossen. Bei der überprüften Öffnung kann es sich um die Entnahmeöffnung, durch die ein Verbrauchsmittel herausgeschoben wird, und/oder um die Einschuböffnung handeln, durch die ein Stößel der Entnahmeeinrichtung in die Kammer eingeführt wird. Da beim Entnehmen eines Verbrauchsmittels sowohl die Siegelfolie der Entnahmeöffnung als auch der Einschuböffnung zerstört wird, läßt sich durch Prüfen der Siegelfolie der Einschuböffnung ebenso wie durch Prüfen der Siegelfolie der Entnahmeöffnung feststellen, ob das Verbrauchsmittel aus der jeweiligen Kammer bereits entnommen wurde. Auf diese Weise kann ein unnötiges Betätigen der Entnahmevorrichtung bei einem Versuch aus einer leeren Kammer ein Verbrauchsmittel zu entnehmen vermieden und so der Stromverbrauch des Analysehandgeräts wesentlich gesenkt werden, insbesondere da der Stromverbrauch eines Analysehandgeräts in der Regel zum größten Teil auf der Entnahmeeinrichtung beruht.

Ein erfolgloses Betätigen der Entnahmeeinrichtung verbraucht aber nicht nur Strom, sondern kostet vor allem auch Zeit. Insbesondere im Fall eines bereits teilweise geleerten Trommelmagazins, das nach einer vorübergehenden Entnahme erneut in ein Analysehandgerät eingelegt wird, kann eine von der Inbetriebnahme des Gerätes bis zum Bereitstellen eines Verbrauchsmittels andauernde Wartezeit bei einem Gerät nach dem Stand der Technik, wegen einer unter Umständen wiederholten erfolglosen Betätigung der Entnahmeeinrichtung, die nacheinander an bereits leeren Kammern einen Entnahmeversuch durchführt, störend lang sein. Vorteilhaft läßt sich diese Wartezeit bei einem erfindungsgemäßes Analysehandgerät wesentlich verkürzen, so daß es sich angenehmer und leichter handhaben läßt.

Um den Stromverbrauch der Entnahmeeinrichtung noch weiter zu reduzieren, kann die Siegelfolie eines Magazins für ein erfindungsgemäßes Analysehandgerät dünner und weniger belastbar als nach dem Stand der Technik sein, so daß sie mit einem geringeren Kraftaufwand durchstoßen werden kann. Die Belastbarkeit der siegelfolie eines Magazins für ein Analysehandgerät wird als Kompromiß widersprüchlicher Anforderungen gewählt. Einerseits muß die Siegelfolie von der Kraft, die von der Entnahmeeinrichtung des Analysehandgeräts aufgebracht werden kann, durchstoßen werden können. Andererseits soll die Siegelfolie auch bei einem unachtsamen Transport möglichst nicht beschädigt werden, da sonst die Gefahr der Verwendung eines durch Umwelteinflüsse beeinträchtigten Verbrauchsmittels besteht. Da bei einem erfindungsgemäßen Analysehandgerät eine beschädigte Siegelfolie erkannt werden kann, ist es zur Erzielung des Vorteils einer weniger Strom verbrauchenden Entnahmeeinrichtung möglich, die Gefahr einer in seltenen Fällen auftretenden Beschädigung der Siegelfolie durch unachtsamen Transport in Kauf zu nehmen.

Ein weiterer Vorteil eines erfindungsgemäßen Analysehandgerätes liegt darin, daß Verbrauchsmittel nach Gebrauch wieder in ihre Kammer des Magazins eingebracht werden können, da eine erneute Ausgabe eines solchen benutzten Verbrauchsmittels zuverlässig vermieden werden kann. Auf diese Weise können alle Verbrauchsmittel eines Magazins nach Gebrauch in dem Magazin remagaziniert und zusammen mit dem Magazin entsorgt werden, so daß ein Benutzer von der Notwendigkeit befreit ist, jedes Verbrauchsmittel nach einer abgeschlossenen Untersuchung mühsam einzeln zu entsorgen. Bei einem erfindungsgemäß ausgebildeten Analysehandgerät wird, anders als nach dem Stand der Technik, erkannt, wenn eine bereits geöffnete Kammer, die ein remagaziniertes Verbrauchsmittel enthält, zur Entnahme bereitgestellt wird. Diese Kammer kann dann übersprungen und das Magazin zur nächsten Kammer weiterbewegt werden.

Die Prüfeinrichtung eines erfindungsgemäßen Analysehandgerätes ermöglicht es auch, eine beschädigte Siegelfolie einer Kammer, in der sich mit ein unbenutztes Verbrauchsmittel befindet, zu erkennen. Bei einer beschädigten Siegelfolie besteht die Gefahr, daß das Verbrauchsmittel durch Feuchtigkeit oder Schmutz beeinträchtigt wurde, so daß eine damit vorgenommene Untersuchung zu einem falschen Ergebnis führen könnte. Durch das Erkennen beschädigter Siegelfolien kann deshalb bei einem erfindungsgemäßen Analysehandgerät die Gefahr einer Verwendung schadhafter Verbrauchsmittel vermindert und damit die Zuverlässigkeit der Untersuchungsergebnisse erhöht werden.

Insbesondere für Anwender, deren Sehkraft eingeschränkt ist, bedeutet dies einen großen Vorteil, da ihnen eine Inspektion der Siegelfolie jeder Kammer eines Magazins vor dem Einlegen desselben in das Analysehandgerät erhebliche Mühe bereitet und deshalb oft unterbleibt.

Für Magazine, deren Kammern nur eine einzige Öffnung aufweisen, die sowohl zur Ausgabe eines Verbrauchsmittels als auch zum Eingriff der Entnahmeeinrichtung dient, genügt eine einzige Prüfeinrichtung, um z. B. eine Verwendung von Verbrauchsmitteln ausschließen zu können, bei denen aufgrund einer beschädigten Siegelfolie die Gefahr einer Beeinträchtigung besteht. Bei Magazinen, deren Kammern sowohl eine Einschub- als auch eine Entnahmeöffnung aufweisen, läßt sich hinsichtlich der ungewollten Beschädigung der Siegelfolien eine völlige Sicherheit nur durch Einsatz zweier Prüfeinrichtungen erreichen. Schon bei Einsatz einer einzigen Prüfeinrichtung an einer der Öffnungen läßt sich aber auch in diesen Fällen das Risiko, ein schadhaftes Verbrauchsmittel zu verwenden um die Hälfte reduzieren oder erkennen, ob aus der jeweiligen Kammer bereits ein Verbrauchsmittel entnommen wurde, unabhängig von dessen eventueller Remagazinierung.

Bei dem Magazin handelt es sich bevorzugt um ein Trommelmagazin, das um seine geometrische Längsachse in dem Analysehandgerät rotierbar ist. Die Erfindng ist aber nicht auf Trommelmagazine beschränkt, sondern ermöglicht beispielsweise auch ein Prüfen der Siegelfolie eines quaderförmigen Magazins, das an der Ausgabeöffnung des Analysehandgerätes vorbeigeschoben wird.

Weitere Einzelheiten und Vorteile der Erfindung werden anhand eines Ausführungsbeispieles unter Bezugnahme auf die beigefügten Figuren erläutert. Die darin dargestellten Besonderheiten können einzeln oder in Kombination verwendet werden, um bevorzugte Ausgestaltungen der Erfindung zu schaffen. Es zeigen:
- Fig. 1: ein Ausführungsbeispiel eines Analysehandgerätes in einer Schrägansicht,
- Fig. 2: das in Fig. 1 gezeigte Ausführungsbeispiel bei geöffnetem Magazinfach mit einem Trommelmagazin und einem Verbrauchsmittel,
- Fig. 3: eine weitere Ansicht des Ausführungsbeispiels,
- Fig. 4: eine Ausschnittvergrößerung von Fig. 3 und
- Fig. 5: den Aufbau der Sensoreinrichtung.

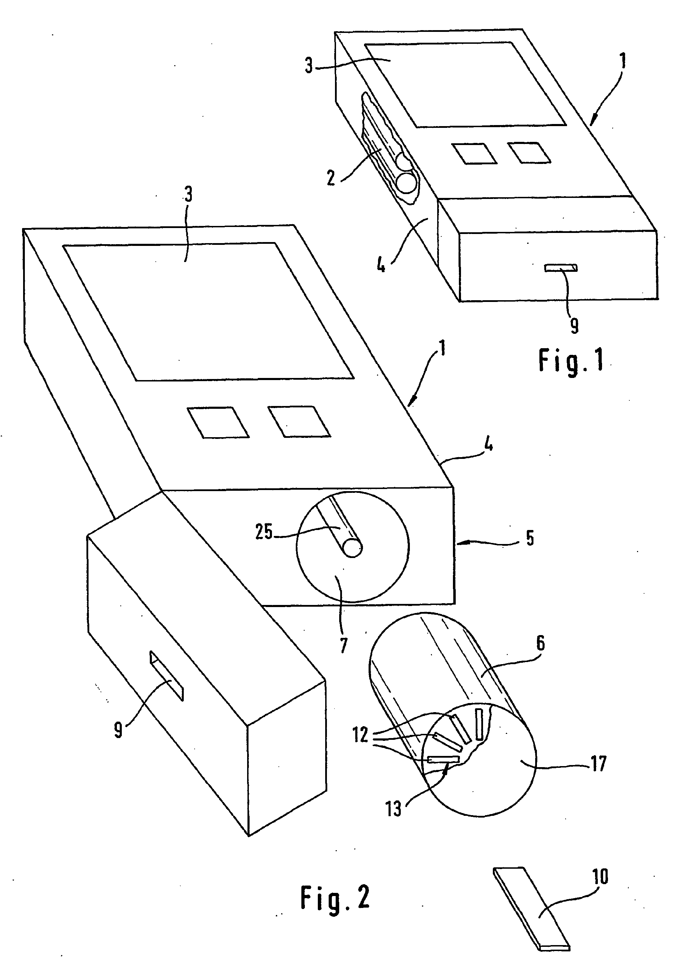

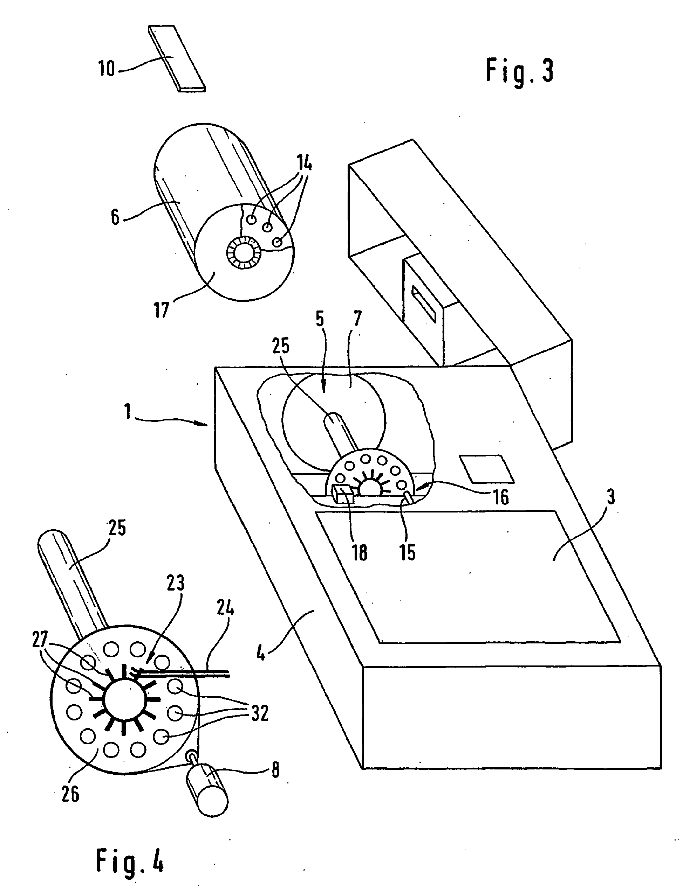

Fig. 1 bis 3 zeigen in verschiedenen Ansichten ein kompaktes, tragbares Analysehandgerät 1 zum Untersuchen eines medizinisch bedeutsamen Bestandteils einer Probe, insbesondere einer biologischen Flüssigkeit, wie beispielsweise Blut, Urin oder Speichel. Das in Fig. 1 gezeigte Analysehandgerät 1 dient zur Bestimmung des Blutglucosegehaltes und weist eine integrierte Stromquelle in Form handelsüblicher Batterien oder Solarzellen auf. Das Ergebnis einer Untersuchung wird mit einer Anzeigeeinrichtung 3, bevorzugt einem stromsparenden Flüssigkristalldisplay angezeigt. Das Analysehandgerät 1 weist ein Gehäuse 4 auf, das eine Ladeöffnung 5 zur Aufnahme eines auswechselbaren Trommelmagazins 6 in ein Magazinfach 7 hat, in dem das Trommelmagazin mittels eines Antriebs schrittweise um seine geometrische Längsachse rotierbar ist. Fig. 1 zeigt das Analysehandgerät mit geschlossener Ladeöffnung 5. Fig. 2 und 3 zeigen das Analysehandgerät 1 bei geöffneter Ladeöffnung 5. In Fig. 3 ist zur besseren Veranschaulichung ein Teil des Gehäuses 4 durchbrochen dargestellt, so daß das Magazinfach 7 einsehbar ist.

In einer Stirnseite weist das Gehäuse 4 eine Ausgabeöffnung 9 für in dem Trommelmagazin 6 gelagerte analytische Verbrauchsmittel 10 auf. Bevorzugt sind diese Verbrauchsmittel 10 als Teststreifen ausgebildet, auf die eine Probe aufgebracht werden kann. Ein im Teststreifen enthaltenes Reagenz reagiert mit einem medizinisch wesentlichen Bestandteil der Probe, so daß das Ergebnis der Reaktion mit einer Analyseeinrichtung des Analysegerätes 1 ausgewertet werden kann. Eine solche Analyseeinrichtung kann beispielsweise ein optischer Sensor sein, der eine Farbänderung eines als Teststreifen ausgebildeten Verbrauchsmittels 10 erfaßt, oder einen elektronischen Sensor enthalten, der eine Leitfähigkeitsänderung der Probe bestimmt.

Das Trommelmagazin 6 hat mehrere ringförmig um seine geometrische Längsachse angeordnete Kammern 12, die analytische Verbrauchsmittel 10 enthalten können. Durch schrittweise Rotation des Trommelmagazins 6 können die Kammern 12 nacheinander in einer Entnahmeposition positioniert werden, so daß die Verbrauchsmittel 10 bei Bedarf aus der jeweiligen Kammer 12 des Trommelmagazins 6 entnommen und durch die Ausgabeöffnung 9 des Gehäuses 4 ausgegeben werden können.

Die Zahl dieser Kammer 12 kann weitgehend beliebig gewählt werden. In der Regel sind 10 bis 100 Kammern 12 zweckmäßig, bevorzugt sind 15 bis 30 Kammern 12 vorhanden. Jede der Kammern 12 weist an einer Stirnseite des Trommelmagazins 6 eine Entnahmeöffnung 13 zum Entnehmen eines Verbrauchsmittels 10 und eine der Entnahmeöffnung 13 gegenüberliegende Einschuböffnung 14 zum Einführen eines Stößels 15 einer Entnahmeeinrichtung 16 auf. Die Einschuböffnungen 14 und die Entnahmeöffnungen 13 sind zum Schutz der Verbrauchsmittel 10 mit einer Siegelfolie 17 verschlossen. Wie in der EP 1 022 565 A2 beschrieben lassen sich mit dem Stößel 15 Verbrauchsmittel 10 zur Verwendung aus den Kammern 12 herausschieben, wobei die Siegelfolie 17 der Einschuböffnung 14 vom Stößel 15 und die Siegelfolie 17 der Entnahmeöffnung 13 von dem Verbrauchsmittel 10 durchstoßen wird.

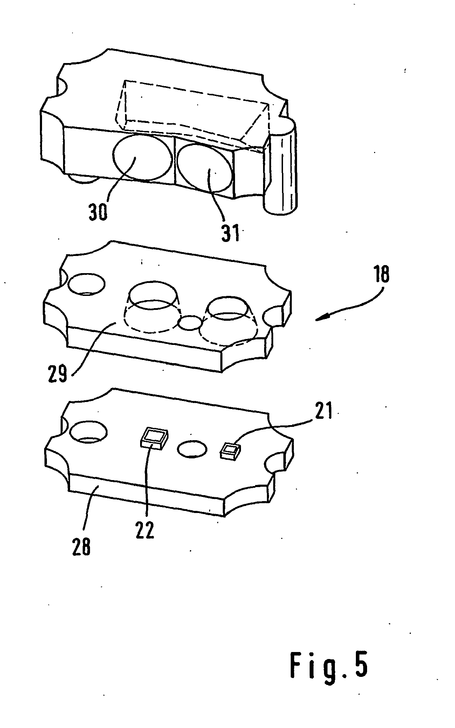

Das Analysehandgerät 1 weist eine anhand der Figuren 4 und 5 im folgenden näher beschriebene Prüfeinrichtung 18 auf, mit der ein Signal erzeugbar ist, das eine Information darüber enthält, ob eine der Einschuböffnungen 14 mit Siegelfolie 17 verschlossen ist. Die Prüfeinrichtung 18 ist mit einer Auswerteeinheit zum Auswerten des Signals verbunden. Bei dem gezeigten Ausführungsbeispiel ist die Prüfeinrichtung 18 derart angeordnet, daß mit ihr die Siegelfolie 17 der Kammer 12 prüfbar ist, die sich in der Entnahmeposition befindet. Es ist aber auch möglich, die Prüfeinrichtung 18 so anzuordnen, daß mit ihr die Siegelfolie 17 einer anderen Kammer 12 prüfbar ist, die beispielsweise einen Rotationsschritt von der Entnahmeposition entfernt ist.

Die Auswerteeinheit umfaßt einen Speicher, in dem für jede der Kammern 12 die Information speicherbar ist, ob die jeweilige Einschuböffnung 14 mit Siegelfolie 17 verschlossen ist. Die Auswerteeinheit ist an die Anzeigeeinrichtung 3 angeschlossen, so daß mittels der Anzeigeeinrichtung 3 eine von der Auswerteeinheit durch Auswertung des mittels der Prüfeinrichtung 18 erzeugten Signals gewonnene Information über die Versiegelung einer oder mehrerer Kammern 12 mit Siegelfolie 17 anzeigbar ist. Insbesondere ist es auch möglich einem Benutzer anzuzeigen, wieviele und/oder welche der Kammern 12 noch ein funktionsfähiges Verbrauchsmittel 10 enthalten.

Die Auswerteeinheit umfaßt eine Steuereinheit zum Steuern des Antriebs 8 und betätigt den Antrieb 8, wenn eine Auswertung des Signals ergibt, daß die Einschuböffnung 14 der zur Entnahme eines Verbrauchsmittels 10 positionierten Kammer 12 nicht mit Siegelfolie 17 verschlossen ist. Daraufhin bewirkt der Antrieb einen Rotationsschritt des Trommelmagazins 6, so daß eine benachbarte Kammer 12 in die Entnahmeposition gebracht wird. Um dabei ein unnötiges Betätigen der Entnahmeeinrichtung 16 zu verhindern, ist die Auswerteeinheit derart an die Entnahmeeinrichtung 16 angeschlossen, daß die Entnahmeeinrichtung 16 nur betätigbar ist, wenn die von der Prüfeinrichtung 18 geprüfte Einschuböffnung 14 der zur Entnahme positionierten Kammer 12 mit Siegelfolie 17 verschlossen ist.

Auf diese Weise wird bei Betätigung der Entnahmeeinrichtung 16 stets automatisch ein Verbrauchsmittel 10 aus einer Kammer 12 entnommen, deren Einschuböffnung 14 mit einer intakten Siegelfolie 17 verschlossen ist. Ein erfolgloses Betätigen der Entnahmeeinrichtung 16 und ein damit verbundenes Einführen des Stößels 15 in eine leere Kammer 12 wird vermieden und der Stromverbrauch des Analysehandgerätes 1 gesenkt. Ein Benutzer muß deshalb die Batterien, welche die Stromquelle 2 bilden, nicht so oft wechseln. Ein weiterer Vorteil ist dabei, daß die mit einem erfolglosen Betätigen der Entnahmeeinrichtung 16 verbundene Wartezeit bei dem beschriebenen Analysehandgerät 1 entfällt. Auch können benutzte Verbrauchsmittel 10 nach einer Untersuchung wieder in ihre Kammer 12 eingebracht und dort aufbewahrt werden, bis alle Verbrauchsmittel 10 eines Trommelmagazins 6 gemeinsam entsorgt werden, da eine erneute Entnahme eines benutzten Verbrauchsmittels 10 ausgeschlossen ist.

Mittels der Prüfeinrichtung 18 kann auch eine beschädigte Siegelfolie einer Einschuböffnung 14 erkannt werden. Um ausschließen zu können, daß Verbrauchsmittel 10 verwendet werden, die aufgrund einer beschädigten Siegelfolie 17 verschmutzt oder beeinträchtigt sind und deshalb unzuverlässige Untersuchungsergebnisse liefern könnten, ist bevorzugt eine weitere Prüfeinrichtung 18 vorhanden, mit der auch die Siegelfolie 17 der Entnahmeöffnung 13 der Kammern 12 überprüft werden kann. Schon mit einer einzigen Prüfeinrichtung 18 läßt sich aber die Gefahr der Verwendung eines aufgrund einer beschädigten Siegelfolie 17 beeinträchtigten Verbrauchsmittels 10 um die Hälfte reduzieren.

Das Analysehandgerät 1 weist einen in einen Öffnungsmechanismus integrierten Schalter auf, bei dessen Betätigüng durch Schließen des Magazinfachs 7 eine Rotation des Trommelmagazins 6 um 360° ausgelöst wird. Mittels der Prüfeinrichtung 18 ermittelt die Auswerteeinheit nach Betätigen des Schalters, wie viele und/oder welche der Kammern 12 (noch) mit Siegelfolie 17 verschlossen sind und zeigt dies mittels der mit ihr verbundenen Anzeigeeinrichtung 3 an. Nach Einlegen eines Trommelmagazins 6 kann ein Anwender durch Betätigen des Schalters leicht erkennen, wie viele Verbrauchsmittel 10 in dem Trommelmagazin 6 noch zur Verfügung stehen oder auch welche der Kammern 12 noch mit Siegelfolie 17 verschlossen sind.

Die in Fig. 4 und insbesondere Fig. 5 gezeigte Prüfeinrichtung 18 weist einen optischen Detektor 21 auf, der beispielsweise als eine PIN-Diode oder ein Fototransistor ausgeführt ist. Die Prüfeinrichtung 18 umfaßt ferner eine Lichtquelle 22 zum Anstrahlen einer der Einschuböffnungen 14 des Trommelmagazins 6. Ist die angestrahlte Einschuböffnung 14 von einer intakten Siegelfolie 17 verschlossen, so wird das von der Lichtquelle 22 ausgestrahlte Licht zumindest teilweise reflektiert und kann dann von dem Detektor 21 detektiert werden. Um ein möglichst starkes Reflexionssignal zu erhalten, weit die Siegelfolie 17 eine metallisch glänzende Oberfläche auf. Beispielsweise kann die Siegelfolie 17 hierfür als eine Aluminiumfolie oder eine mit metallbeschichtete Kunststoffolie ausgeführt sein. Ist die Siegelfolie 17 beschädigt, so wird weniger Licht zum Detektor 21 reflektiert, so daß eine intakte von einer beschädigten oder durchstoßenen Siegelfolie 17 und damit eine geöffnete von einer verschlossenen Kammer 12 unterschieden werden kann.

Zum Feststellen ob sich eine der Kammern 12 in der Entnahmeposition befindet weist das Analysehandgerät 1 in Fig. 4 gezeigte Positionserkennungsmittel 23 auf. Die Positionserkennungsmittel 23 umfassen bevorzugt einen Schleifkontakt 24, der beispielsweise als ein Kontaktfinger ausgebildet sein kann. Der Schleifkontakt 24 ist gegenüber dem Trommelmagazin 6 ortsfest angeordnet und schleift an dem Trommelmagazin 6 oder einem zusammen mit dem Trommelmagazin 6 rotierenden Bauteil des Analysehandgerätes 1 entlang. Bei dem gezeigten Ausführungsbeispiel ist das Trommelmagazin 6 auf einem Dorn 25 gelagert, der sich zusammen mit dem Trommelmagazin 6 dreht und mit einem Indexrad 26 verbunden ist.

Das Indexrad 26 kann wahlweise an der Stirnseite des Trommelmagazins 6 mit den Einschuböffnungen 14 oder auch an dessen gegenüberliegender Stirnseite angeordnet sein. Das in Fig. 4 gezeigt Indexrad 26 weist fluchtend mit den Einschuböffnungen 14 positionierte Durchbrüche 32 auf, durch die Licht der Sensoreinrichtung 18 und der Stößel 15 hindurchtreten können. Das Indexrad 26 ist mit ringförmig angeordneten, voneinander beabstandeten Kontaktfeldern 27 versehen, deren Anzahl der Anzahl der Kammern 12 des Trommelmagazins 6 entspricht. Bei einer Rotation des Trommelmagazins 6 kommen diese Kontaktfelder 27 nacheinander mit dem als Kontaktfinger ausgebildeten Schleifkontakt 24 in Kontakt, wobei jedesmal ein Stromkreis geschlossen und somit ein Signal erzeugt wird. Durch Zählen dieser Signale kann die Auswerteeinheit feststellen, wann das Trommelmagazin 6 eine volle 360°-Drehung vollendet hat und welche der Kammern 12 in der Entnahmeposition positioniert ist.

Die Kontaktfelder 27 können dabei wahlweise so angeordnet sein, daß sich eine der Kammern 12 in der Entnahmeposition befindet, wenn eines der Kontaktfelder 27 mit dem als Kontaktfinger ausgebildeten Schleifkontakt 24 in Kontakt steht oder umgekehrt. Alternativ ist es auch möglich, den Schleifkontakt 24 an dem Indexrad 26 und die mit ihm zusammenwirkenden Kontaktfelder 27 ortsfest in dem Magazinfach 7 anzuordnen.

Bevorzugt wird durch das von den Positionserkennungsmitteln 23 erzeugte Signal auch die Prüfeinrichtung 18 aktiviert. Dies läßt sich am einfachsten dadurch erreichen, daß die Prüfeinrichtung 18 in einem Stromkreis angeordnet ist, der durch Kontakt des Schleifkontaktes 24 mit einem der Kontaktfelder 27 geschlossen wird. Das durch die Positionserkennungsmittel 23 gewonnene Signal wird bevorzugt auch zur Steuerung des als Elektromotor ausgeführten Antriebs 8 für die Rotation des Trommelmagazins 6 genutzt, so daß der Antrieb 8 automatisch stoppt, wenn eine der Kammern 12 in die Entnahmeposition gelangt ist.

Fig. 5 veranschaulicht in einer Explosionsdarstellung den Aufbau der Prüfeinrichtung 18. Die bevorzugt als ein LED ausgeführte Lichtquelle 22 ist zusammen mit dem als Fototransistor ausgeführten Detektor 21 auf einer gemeinsamen Platine 28 angeordnet, was einen platzsparenden und kostengünstigen Aufbau ermöglicht. Um das Licht der Lichtquelle 22 möglichst effizient zu nutzen und ein möglichst gutes Signal-Rauschverhältnis zu erreichen, weist die Prüfeinrichtung 18 eine Optik auf, die eine Blende 29, eine erste Linse 30 und eine zweite Linse 31 umfaßt. Vor der Platine 28 befindet sich die Blende 29, die störendes Streulicht minimiert und so die Sensitivität des Detektors 21 erhöht. Die erste Linse 30 führt der zu prüfenden Einschuböffnung 14 Licht der Lichtquelle 22 zu, so daß das von der Lichtquelle 22 ausgesandte Licht möglichst effizient ausgenutzt wird. Damit eine schadhafte Siegelfolie 17 möglichst zuverlässig erkannt werden kann, sollte die zu prüfende Siegelfolie 17 möglichst völlflächig ausgeleuchtet werden und das von der Lichtquelle 22 ausgesandte Licht nicht lediglich in einen kleinen Bereich der Einschuböffnung 14 fokussiert werden. Eine zweite Linse 31 fokussiert das von der Siegelfolie 17 reflektierte Licht auf den Detektor 21. Ein einstückiges Spritzgußteil bildet sowohl die erste Linse 30 als auch die zweite Linse 31 aus, was die Montage der Prüfeinrichtung 18 erleichtert und vorteilhaft kostengünstig ist.

### Bezugszeichenliste

- 1: Analysehandgerät
- 2: Stromquelle
- 3: Anzeigeeinrichtung
- 4: Gehäuse
- 5: Ladeöffnung
- 6: Trommelmagazin
- 7: Magazinfach
- 9: Ausgabeöffnung
- 10: Verbrauchsmittel
- 12: Kammer
- 13: Entnahmeöffnung
- 14: Einschuböffnung
- 15: Stößel
- 16: Entnahmeeinrichtung
- 17: Siegelfolie
- 18: Prüfeinrichtung
- 21: Detektor
- 22: Lichtquelle
- 23: Positionserkennungsmittel
- 24: Schleifkontakt
- 25: Dorn
- 26: Indexrad
- 27: Kontaktfelder
- 28: Platine
- 29: Blende
- 30: erste Linse
- 31: zweite Linse
- 32: Durchbrüche

## Patentansprüche

1. Analysehandgerät zum Untersuchen einer Probe, insbesondere einer biologischen Flüssigkeit, hinsichtlich eines medizinisch bedeutsamen Bestandteils, umfassend
eine Analyseeinrichtung,
eine Anzeigeeinrichtung (3),
ein Gehäuse (4), das eine Ladeöffnung (5) zum Aufnehmen eines auswechselbaren Magazins (6) mit mehreren Kammern (12) aufweist, die analytische Verbrauchsmittel (10), insbesondere Teststreifen, enthalten können und jeweils eine Öffnung (13,14) an wenigstens einer Stirnseite des Magazins (6) aufweisen, die jeweils mit einer Siegelfolie (17) verschlossen sein kann,
eine Entnahmeeinrichtung (16) zum Entnehmen eines der analytischen Verbraüchsmittel (10) aus dem Magazin (6), mittels der eines der Verbrauchsmittel (10) aus einer der Kammern (12) des Magazins (6) heraus befördert werden kann, wobei die von der Siegelfolie (17) verschlossene Öffnung (13,14) geöffnet wird, und
einen Antrieb, der zum.Positionieren einer Kammer (12) in eine Entnahmeposition, in der mittels der Entnahmeeinrichtung (16) ein Verbrauchsmittel (10) aus der Kammer (12) entnehmbar ist, eine Bewegung des Magazins (6) ermöglicht,
**dadurch gekennzeichnet, daß**
das Analysehandgerät (1) eine Prüfeinrichtung (18) aufweist, mit der ein Signal erzeugbar ist, das eine Information darüber enthält, ob die Öffnung (13,14) einer der Kammern (12) mit Siegelfolie (17) verschlossen ist, wobei die Prüfeinrichtung (18) mit einer Auswerteeinheit zum Auswerten des Signals verbunden ist.

2. Analysehandgerät nach Anspruch 1, **dadurch gekennzeichnet, daß** die Prüfeinrichtung (18) derart angeordnet ist, daß mit ihr die Siegelfolie (17) der Kammer (12) prüfbar ist, die sich in der Entnahmeposition befindet.

3. Analysehandgerät nach einem der vorhergehenden Ansprüche, **dadurch gekennzeichnet, daß** die Auswerteeinheit einen Speicher aufweist, in dem für mindestens eine der Kammern (12), vorzugsweise für jede der Kammern (12), die Information, ob deren Öffnung (13,14) mit Siegelfolie (17) verschlossen ist, speicherbar ist.

4. Analysehandgerät nach einem der vorhergehenden Ansprüche, **dadurch gekennzeichnet, daß** die Auswerteeinheit derart an die Entnahmeeinrichtung (16) angeschlossen ist, daß die Entnahmeeinrichtung nur betätigbar ist, wenn die von der Prüfeinrichtung (18) geprüfte Öffnung (13,14) der Kammer (12), die sich in der Entnahmeposition befindet, mit Siegelfolie verschlossen ist.

5. Analysehandgerät nach einem der vorhergehenden Ansprüche, **dadurch gekennzeichnet, daß** die Auswerteeinheit eine Steuereinheit zum Steuern des Antriebs umfaßt, wobei die Auswerteeinheit (den Antrieb betätigt, wenn die Öffnung (13,14) der Kammer (12) , die sich in der Entnahmeposition befindet, nicht mit Siegelfolie (17) verschlossen ist.

6. Analysehandgerät nach einem der vorhergehenden Ansprüche, **dadurch gekennzeichnet, daß** das Magazin (6) ein Trommelmagazin ist und bei einer Rotation des Magazins (6) um 360° mittels der Prüfeinrichtung (18) und der Auswerteeinheit feststellbar ist, wie viele und/oder welche der Kammern (12) mit Siegelfolie (17) verschlossen sind.

7. Analysehandgerät nach Anspruch 6, **dadurch gekennzeichnet, daß** es einen Schalter aufweist, nach dessen Betätigung die Auswerteeinheit automatisch ermittelt, wie viele und/oder welche der Kammern (12) mit Siegelfolie (17) verschlossen sind.

8. Analysehandgerät nach einem der vorhergehenden Ansprüche, **dadurch gekennzeichnet, daß** die Auswerteeinheit an die Anzeigeeinrichtung (3) angeschlossen ist und mittels der Anzeigeeinrichtung (3) eine von der Auswerteeinheit durch Auswertung des mittels der Prüfeinrichtung (18) erzeugten Signals gewonnene Information über die Versiegelung einer oder mehrerer Kammern (12) des Magazins (6) mit Siegelfolie (17) anzeigbar ist.

9. Analysehandgerät nach Anspruch 8, **dadurch gekennzeichnet, daß** mittels der Anzeigeeinrichtung (3) anzeigbar ist, wie viele und/oder welche der Kammern (12) mit Siegelfolie (17) verschlossen sind.

10. Analysehandgerät nach einem der vorhergehenden Ansprüche, **dadurch gekennzeichnet, daß** die Prüfeinrichtung (18) einen optischen Detektor (21) aufweist.

11. Analysehandgerät nach Anspruch 10, **dadurch gekennzeichnet, daß** der Detektor (21) als ein Fototransistor ausgebildet ist.

12. Analysehandgerät nach einem der vorhergehenden Ansprüche, **dadurch gekennzeichnet, daß** die Prüfeinrichtung (18) eine Lichtquelle (22) zum Anstrahlen einer der Öffnungen (13,14) des Magazins (6) aufweist.

13. Analysehandgerät nach Anspruch 12, **dadurch gekennzeichnet, daß** die Lichtquelle (22) als ein LED ausgebildet ist.

14. Analysehandgerät nach einem der vorhergehenden Ansprüche, **dadurch gekennzeichnet, daß** die Siegelfolie (17) eine metallisch glänzende Oberfläche aufweist.

15. Analysehandgerät nach einem der Ansprüche 10 bis 14, **dadurch gekennzeichnet, daß** der Detektor (21) und die Lichtquelle (22) auf einer gemeinsamen Platine (28) angeordnet sind.

16. Analysehandgerät nach einem der Ansprüche 10 bis 15, **dadurch gekennzeichnet, daß** die Prüfeinrichtung (18) eine Optik (29,30,31) zum Zuführen von Licht der Lichtquelle (22) auf eine der Öffnungen (13,14) und/oder zum Fokussieren von der Siegelfolie (17) reflektiertem Licht auf den Detektor (21) aufweist.

17. Analysehandgerät nach Anspruch 16, **dadurch gekennzeichnet, daß** die Optik (30,31) eine erste Linse (30) zum Zuführen-von Licht der Lichtquelle (22) auf eine der Öffnungen (13) und eine zweite Linse (31) zum Fokusieren des von der Siegelfolie (17) reflektierten Lichtes auf den Detektor (21) aufweist.

18. Analysehandgerät nach Anspruch 17, **dadurch gekennzeichnet, daß** die Prüfeinrichtung (18) ein einstückiges Spritzgußteil umfaßt, in dem die erste (30) und zweite (31) Linse ausgebildet sind.

19. Analysehandgerät nach einem der vorhergehenden Ansprüche, **dadurch gekennzeichnet, daß** Positionserkennungsmittel (23,24,27) zum Feststellen, ob eine der Kammern (12) in der Entnahmeposition positioniert ist, vorhanden sind und die Prüfeinrichtung (18) durch die Positionserkennungsmittel (23,24,27) betätigbar ist.

## Claims

1. Hand-held analytical device for analysing a sample, in particular a biological fluid, for a medically significant component, comprising
an analytical facility,
a display facility (3),
a housing (4) comprising a loading opening (5) for receiving a replaceable cartridge (6) having multiple chambers (12) that may contain analytical consumables (10), in particular test strips, each chamber (12) comprising an opening (13,14) at at least one front face of the cartridge (6), wherein each opening (13,14) may be sealed by a sealing foil (17),
a removal facility (16) for removing one of the analytical consumables (10) from the cartridge (6), wherein the removal facility (16) can be used to remove one of the consumables (10) from one of the chambers (12) of the cartridge (6), in the process of which the opening (13,14) sealed by the sealing foil (17) is opened, and
a drive allowing the cartridge (6) to be moved in order to position one chamber (12) in a removal position, in which a consumable (10) can be removed from the chamber (12) by means of the removal facility (16),
**characterised in that**
the hand-held analytical device (1) comprises a testing facility (18) that can be used to generate a signal containing an information regarding whether or not the opening (13,14) of one of the chambers (12) is sealed by sealing foil (17), whereby the testing facility (18) is connected to an analytical facility for analysis of the signal.

2. Hand-held analytical device according to claim 1, **characterised in that** the testing facility (18) is arranged such that it can be used to test the sealing foil (17) of the chamber (12) residing in the removal position.

3. Hand-held analytical device according to any one of the preceding claims, **characterised in that** the analytical unit comprises a memory, in which the information regarding the sealing of the respective opening (13,14) by sealing foil (17) can be stored for at least one of the chambers (12), preferably for each of the chambers (12).

4. Hand-held analytical device according to any one of the preceding claims, **characterised in that** the analytical unit is connected to the removal facility (16) such that the removal facility can be actuated only if the opening (13,14) of the chamber (12) residing in the removal position that is tested by the testing facility (18) is sealed by sealing foil.

5. Hand-held analytical device according to any one of the preceding claims, **characterised in that** the analytical unit comprises a control unit for controlling the drive, whereby the analytical unit actuates the drive when the opening (13,14) of the chamber (12) residing in the removal position is not sealed by sealing foil (17).

6. Hand-held analytical device according to any one of the preceding claims, **characterised in that** the cartridge (6) is a drum cartridge, and, upon a rotation of the cartridge (6) by 360°, the testing facility (18) and the analytical unit can be used to determine how many and/or which of the chambers (12) are sealed by sealing foil (17).

7. Hand-held analytical device according to claim 6, **characterised in that** it comprises a switch after the actuation of which the analytical unit automatically determines how many and/or which of the chambers (12) are sealed by sealing foil (17).

8. Hand-held analytical device according to any one of the preceding claims, **characterised in that** the analytical unit is connected to the display facility (3) and the display facility (3) can be used to display an information regarding the sealing of one or multiple chambers (12) of the cartridge (6) by sealing foil (17) that is obtained by the analytical unit by analysing the signal generated by the testing facility (18).

9. Hand-held analytical device according to claim 8, **characterised in that** the display facility (3) can be used to display how many and/or which of the chambers (12) are sealed by sealing foil (17).

10. Hand-held analytical device according to any one of the preceding claims, **characterised in that** the testing facility (18) comprises an optical detector (21).

11. Hand-held analytical device according to claim 10, **characterised in that** the detector (21) is provided in the form of a phototransistor.

12. Hand-held analytical device according to any one of the preceding claims, **characterised in that** the testing facility (18) comprises a light source (22) for illuminating one of the openings (13,14) of the cartridge (6).

13. Hand-held analytical device according to claim 12, **characterised in that** the light source (22) is provided in the form of an LED.

14. Hand-held analytical device according to any one of the preceding claims, **characterised in that** the sealing foil (17) comprises a metallically glossy surface.

15. Hand-held analytical device according to any one of the claims 10 to 14, **characterised in that** the detector (21) and the light source (22) are arranged jointly on a common plate (28).

16. Hand-held analytical device according to any one of the claims 10 to 15, **characterised in that** the testing facility (18) comprises an optical system (29,30,31) for guiding light of the light source (22) onto one of the openings (13,14) and/or for focusing of light reflected by the sealing foil (17) onto the detector (21).

17. Hand-held analytical device according to claim 16, **characterised in that** the optical system (30,31) comprises a first lens (30) for guiding light of the light source (22) onto one of the openings (13) and a second lens (31) for focusing of the light reflected by the sealing foil (17) onto the detector (21).

18. Hand-held analytical device according to claim 17, **characterised in that** the testing facility (18) comprises a one-piece injection moulded part in which the first (30) and second (31) lens are provided.

19. Hand-held analytical device according to any one of the preceding claims, **characterised in that** position recognition means (23,24,27) are present for determining whether or not one of the chambers (12) is positioned in the removal position, and **in that** the testing facility (18) can be actuated by the position recognition means (23,24,27).

## Revendications

1. Appareil manuel d'analyse destiné à l'étude d'un échantillon, en particulier d'un liquide biologique, par rapport à un élément significatif sur le plan médical, comprenant
un système d'analyse,
un système d'affichage (3),
un boîtier (4) qui présente une ouverture de chargement (5) destinée à accueillir un chargeur remplaçable (6) avec plusieurs compartiments, qui peuvent contenir des consommables analytiques (10), en particulier des bandes de test, et présentent respectivement une ouverture (13, 14) au niveau au moins d'une face avant du chargeur (6), qui peut respectivement être fermée avec un film de scellage (17),
un système d'extraction (16) destiné à extraire l'un des consommables analytiques (10) du chargeur (6), au moyen duquel l'un des consommables (10) peut être retiré de l'un des compartiments (12) du chargeur (6), l'ouverture (13, 14) fermée par le film de scellage (17) étant ouverte, et
un mécanisme d'entraînement qui, pour le positionnement d'un compartiment (12) dans une position d'extraction, dans laquelle un consommable (10) peut être extrait du compartiment (12) au moyen du système d'extraction (16), permet un déplacement du chargeur (6),
**caractérisé en ce que**
l'appareil manuel d'analyse (1) présente un système de vérification (18), avec lequel un signal peut être généré, qui contient des informations indiquant si l'ouverture (13, 14) d'un des compartiments (12) est fermée par un film de scellage (17), le système de vérification (18) étant relié à une unité d'évaluation destinée à évaluer le signal.

2. Appareil manuel d'analyse selon la revendication 1, **caractérisé en ce que** le système de vérification (18) est agencé de telle sorte qu'avec lui le film de scellage (17) du compartiment (12) qui se trouve dans la position d'extraction peut être vérifié.

3. Appareil manuel d'analyse selon l'une des revendications précédentes, **caractérisé en ce que** l'unité d'évaluation présente une mémoire, dans laquelle pour au moins l'un des compartiments (12), de préférence pour chacun des compartiments (12), l'information indiquant si leur ouverture (13, 14) est fermée avec le film de scellage (17) est mémorisable.

4. Appareil manuel d'analyse selon l'une des revendications précédentes, **caractérisé en ce que** l'unité d'évaluation est raccordée au système d'extraction (16) de telle sorte que le système d'extraction n'est actionnable que si l'ouverture (13, 14) du compartiment (12) vérifiée par le système de vérification (18) et qui se trouve dans la position d'extraction, est fermée avec le film de scellage.

5. Appareil manuel d'analyse selon l'une des revendications précédentes, **caractérisé en ce que** l'unité d'évaluation comprend une unité de commande destinée à commander le mécanisme d'entraînement, l'unité d'évaluation actionnant le mécanisme d'entraînement si l'ouverture (13, 14) du compartiment (12) qui se trouve dans la position d'extraction n'est pas fermée avec le film de scellage (17).

6. Appareil manuel d'analyse selon l'une des revendications précédentes, **caractérisé en ce que** le chargeur (6) est un chargeur tambour et lors d'une rotation du chargeur (6) de 360° au moyen du système de vérification (18) et de l'unité d'évaluation on peut détecter combien et/ou lesquels des compartiments (12) sont fermés avec le film de scellage (17).

7. Appareil manuel d'analyse selon la revendication 6, **caractérisé en ce qu'**il présente un commutateur, après l'activation duquel l'unité d'évaluation détermine automatiquement combien et/ou lesquels des compartiments (12) sont fermés avec le film de scellage (17).

8. Appareil manuel d'analyse selon l'une des revendications précédentes, **caractérisé en ce que** l'unité d'évaluation est raccordée au système d'affichage (3) et au moyen du système d'affichage (8) une information, obtenue par l'unité d'évaluation via l'évaluation du signal généré au moyen du système de vérification (18), concernant le scellage d'un ou plusieurs compartiments (12) du chargeur (6) avec le film de scellage (17) est affichable.

9. Appareil manuel d'analyse selon la revendication 8, **caractérisé en ce qu'**au moyen du dispositif d'affichage (3) on peut afficher combien et/ou lesquels des compartiments (12) sont fermés avec le film de scellage (17).

10. Appareil manuel d'analyse selon l'une des revendications précédentes, **caractérisé en ce que** le système de vérification (18) présente un détecteur optique (21).

11. Appareil manuel d'analyse selon la revendication 10, **caractérisé en ce que** le détecteur (21) est configuré en tant que phototransistor.

12. Appareil manuel d'analyse selon l'une des revendications précédentes, **caractérisé en ce que** le système de vérification (18) présente une source de lumière (22) destinée à illuminer l'une des ouvertures (13, 14) du chargeur (6).

13. Appareil manuel d'analyse selon la revendication 12, **caractérisé en ce que** la source de lumière (22) est configurée en tant que LED.

14. Appareil manuel d'analyse selon l'une des revendications précédentes, **caractérisé en ce que** le film de scellage (17) présente une surface au brillant métallique.

15. Appareil manuel d'analyse selon l'une des revendications 10 à 14, **caractérisé en ce que** le détecteur (21) et la source de lumière (22) sont agencés sur une platine commune (28).

16. Appareil manuel d'analyse selon l'une des revendications 10 à 15, **caractérisé en ce que** le dispositif de vérification (18) présente une optique (29, 30, 31) destinée à amener la lumière de la source de lumière (22) à l'une des ouvertures (13, 14) et/ou destinée à focaliser la lumière réfléchie par le film de scellage (17) sur le détecteur (21).

17. Appareil manuel d'analyse selon la revendication 16, **caractérisé en ce que** l'optique (30, 31) présente une première lentille (30) destinée à amener la lumière de la source de lumière (22) à l'une des ouvertures (13) et une seconde lentille (31) destinée à focaliser la lumière réfléchie par le film de scellage (17) sur le détecteur (21).

18. Appareil manuel d'analyse selon la revendication 17, **caractérisé en ce que** le système de vérification (18) comprend une pièce moulée par injection d'un seul tenant, dans laquelle la première (30) et la seconde (31) lentille sont configurées.

19. Appareil manuel d'analyse selon l'une des revendications précédentes, **caractérisé en ce que** des moyens de reconnaissance de position (23, 24, 27) destinés à détecter si l'un des compartiments (12) est positionné dans la position d'extraction sont présents et le système de vérification (18) est actionnable via les moyens de reconnaissance de position (23, 24, 27).
